# FASCICULE DE BREVET EUROPEEN

(11) **EP 3 628 267 B1**
(45) Date de publication et mention de la délivrance du brevet: **09.09.2020**
(21) Numéro de dépôt: 19198026.7
(22) Date de dépôt: 18.09.2019
(51) Int. Cl.: A61C 5/00, A61C 13/087, A61C 13/00, A61L 27/34, A61L 27/56, A61L 27/16, A61L 27/44, A61L 27/50

(54) **DISPOSITIF DENTAIRE EXTRACORONAIRE**
EXTRAKORONARE DENTALVORRICHTUNG
EXTRACORONAL DENTAL DEVICE

(30) Priorité: 25.09.2018 EP 18306237
(43) Date de publication de la demande: 01.04.2020
(73) Titulaire: Hasson, Jean-Nicolas, 68100 Mulhouse (FR)
(72) Inventeur: Hasson, Jean-Nicolas, 68100 Mulhouse (FR)
(74) Mandataire: Kessler, Marc

(56) Documents cités:
- EP-A2- 1 243 231
- FR-A1- 2 336 115
- JP-A- 2014 132 943
- US-A1- 2005 079 470
- US-A1- 2018 133 374

## Description

### Objet de l'invention

La présente invention se rapporte à un dispositif dentaire extra-coronaire.

### Etat de la technique

Dans le domaine de l'odontologie prothétique, il est connu d'utiliser des couronnes prothétiques en remplacement d'une dent abîmée, mais dont la racine a été préservée. De telles couronnes peuvent être associées à un faux moignon, encore appelé « inlay-core », ancré dans la racine de la dent.

Le document EP 1 243 231 décrit la fabrication d'une telle couronne prothétique par impression 3D, couche par couche, à partir d'un matériau en poudre se liant en couche ou d'un matériau durcissable à l'air. Le matériau peut être polymérisable, à base par exemple de monomère (méth)acrylate ou co-polymère méthacrylate et acrylique ou polyuréthane, et peut comprendre des charges organiques ou inorganique, ou fibres de verre.

Dans le cadre de tels traitements implantaires, mais également de traitements parodontaux, sont également connus les dispositifs dentaires extra-coronaires, qui ne sont pas des couronnes prothétiques, qu'elles soient permanentes ou définitives. Il s'agit de dispositifs temporaires, semi permanents ou permanents, liant de façon externe la couronne d'au moins une dent.

Il peut s'agir d'attelles de contention extra-coronaires, généralement utilisées pour limiter ou stopper un phénomène de mobilité dentaire, ou un déchaussement dentaire, encore appelé parodontite, ou pour stabiliser des dents après un traitement orthodontique. Il peut également s'agir de bridges extra-coronaires qui sont, quant à eux, utilisés après l'extraction d'une dent et avant la mise en charge d'une couronne prothétique sur un implant, afin de fournir rapidement au patient une dent de remplacement, lui procurant ainsi confort et esthétisme.

Les attelles extra-coronaires sont habituellement faites, ou comprennent, un ou plusieurs fils de ligature, une grille, une plaque métallique ou des bandes de ligatures, qui sont fixé(e)s, au niveau d'au moins une des faces d'au moins une dent solidement implantée, dans le but de les immobiliser l'une par rapport à l'autre. Généralement, ces attelles sont disposées sur les faces linguales ou palatines des dents, ou dans des rainures linguales ou palatines pratiquées dans les dents, et maintenues fixées à l'aide d'un adhésif ou ciment dentaire.

Par exemple, le document US 4 433 960 décrit une attelle de contention extra-coronaire, qui est une bande malléable, faite de métal ou de résine, comprenant des perforations, et, sur l'une de ces faces, un adhésif sensible à la pression.

Le document US 2012028221 décrit une attelle extra-coronaire faite de zircone et d'alumine, dont la surface devant adhérer aux dents reprend la forme de la face linguale des dents à stabiliser. Ce document décrit également une méthode d'obtention d'une telle attelle qui comprend l'étape d'une prise d'une empreinte des dents à stabiliser par l'emploi d'alginate, d'élaboration d'un modèle en plâtre des dents, de modéliser informatiquement en trois dimensions les dents afin de générer un modèle virtuel de la dentition du patient, de définir les dimensions de l'attelle, et enfin de former l'attelle, par fraisage, à partir d'un bloc de matériau à base de zircone et d'alumine.

Une telle attelle présente de nombreux inconvénients. Tous d'abord, elle est très onéreuse à produire. Ensuite, elle présente une épaisseur importante qui peut gêner l'utilisateur. Enfin elle présente des problèmes d'adhésion aux dents sur lesquelles elle doit être installée. Non seulement un agent de mordançage, de l'acide phosphorique, doit être appliqué sur les dents saines, ce qui les fragilise, mais de plus, le ciment d'adhésion doit être un ciment fait à base des mêmes composants que ceux de l'attelle, ce qui empêche l'usage des ciments ou colles dentaires classiques, habituellement utilisées pour les attelles dentaires, et peut provoquer des fractures ou des problèmes lors du descellement de l'attelle.

Pour ce qui concerne les bridges extra-coronaires, ces derniers comprennent généralement une gouttière thermoformée, habituellement faite en polyuréthane et polyéthylène, et une couronne prothétique disposée dans la gouttière afin de correspondre à l'emplacement de la dent extraite. L'avantage de l'emploi d'un tel bridge réside dans le fait qu'aucune pression n'est exercée à l'emplacement de l'extraction dentaire. Toutefois, pour des raisons de confort, l'épaisseur de la gouttière est réduite à une épaisseur minimale, ce qui la rend fragile et limite son utilisation à des traitements de courte durée. Ensuite, son port immédiat n'est pas possible avant la coagulation complète de l'emplacement de la chirurgie, sous peine de voir s'infiltrer du sang entre la gouttière et l'élément prothétique, ce qui la rend particulièrement inesthétique.

### Buts de l'invention

La présente invention vise à fournir un dispositif dentaire et une méthode de réalisation d'un tel dispositif, qui ne présentent pas les inconvénients de l'état de la technique.

La présente invention vise à fournir une alternative aux solutions de l'état de la technique existantes.

La présente invention vise à fournir un dispositif de dentaire extra-coronaire temporaire, qui soit bon marché, esthétique, d'une rigidité améliorée et permettant une adhérence facilitée et améliorée aux dents.

### Résumé de l'invention

La présente invention porte sur un dispositif dentaire extra-coronaire comprenant un corps monobloc fait d'un polymère ou co-polymère, obtenu en polymérisant une composition comprenant un monomère polymérisable à base d'acrylate, le dispositif dentaire comprenant une porosité de surface, sur une épaisseur comprise entre 0,1 et 0,8 mm, la porosité comprenant des pores, dont la taille est comprise entre 10 *µ*m et 800 *µ*m, sur ladite épaisseur.

Selon des modes particuliers de l'invention, le dispositif selon l'invention comprend au moins une, ou une combinaison quelconque appropriée, des caractéristiques suivantes :
- la porosité de surface comprend des pores dont la taille est comprise entre 10 *µ*m et 20 *µ*m, 10 *µ*m et 200 *µ*m ou 200 *µ*m et 800 *µ*m,
- la porosité de surface est comprise entre 20 à 60%,
- la porosité de surface est constitué par la surface externe du corps du dispositif dentaire, ou est constituée par un revêtement poreux recouvrant la surface externe du corps,
- le corps est fait d'un polymère ou co-polymère, obtenu en polymérisant une composition comprenant un monomère polymérisable choisis parmi l'acide acrylique, de l'acide méthacrylique, du méthacrylate de méthyle, et leur mélange,
- le revêtement poreux est fait d'un polymère ou co-polymère, obtenu en polymérisant une composition comprenant un monomère polymérisable choisi parmi l'acide acrylique, de l'acide méthacrylique, du méthacrylate de méthyle, et leur mélange,
- le dispositif comprend en outre un ciment d'adhésion recouvrant tout ou partie de la porosité de surface,
- le dispositif a une forme complémentaire et des dimensions correspondant, à l'aspect lingual ou palatine, et/ou la surface occlusale, et/ou la surface vestibulaire de la ou des dents sur laquelle ou lesquelles il va se fixer,

Le dispositif dentaire selon l'invention est utilisé pour prévenir, traiter ou stabiliser un déchaussement dentaire, ou pour participer temporairement à la mise en place d'un implant.

La présente invention porte aussi sur une méthode d'obtention d'un dispositif dentaire extra-coronaire comprenant des porosités de surface, la méthode comprenant l'étape de prendre, ou réaliser, un corps extra-coronaire monobloc à base d'un polymère ou copolymère thermoplastique, comprenant une porosité de surface sur une épaisseur comprise entre environ 0,1 et 0,8 mm, comprenant des pores, dont la taille est comprise entre 10 *µ*m et 800 *µ*m, sur ladite épaisseur.

Selon des modes particuliers de l'invention, la méthode selon l'invention comprend au moins une, ou une combinaison quelconque appropriée, des caractéristiques suivantes :
- l'étape de réalisation du corps extra-coronaire comprend les étapes de prendre, ou réaliser, une composition pour la réalisation du corps, comprenant un ou plusieurs monomères polymérisables à base d'acrylate, et comprenant une ou plusieurs charges porogènes, ou ne comprenant pas de charges porogènes, de prendre, ou réaliser, une composition pour la réalisation d'un revêtement poreux comprenant un ou plusieurs monomères polymérisables à base d'acrylate et comprenant une ou plusieurs charges porogènes, de polymériser la composition pour la réalisation du corps, comprenant des charges porogènes, afin d'obtenir un corps dont la surface extérieur est poreuse, ou polymériser la composition pour la réalisation du corps ne comprenant pas de charges porogènes, afin d'obtenir un corps dont la surface extérieure n'est pas poreuse, de recouvrir à l'aide de la composition pour la réalisation d'un revêtement poreux, le corps dont la surface extérieure est poreuse, ou le corps dont la surface extérieure n'est pas poreuse, puis polymériser la composition pour la réalisation d'un revêtement poreux,
- la méthode comprend en outre une étape d'accentuer la porosité de la surface extérieure du corps, ou celle du revêtement poreux, par une hydrolyse de la surface extérieure, ou par une étape d'abrasion de la surface extérieure à l'aide d'une solution à base d'érythriol, d'une composition à base d'Alumine (Al₂O₃) ou à base de carbonate et/ou bicarbonate d'un sel d'un métal alcalin ou alcalinoterreux, de préférence d'un métal choisi parmi le sodium, le potassium ou le calcium, le métal pouvant être identique ou différent entre le carbonate et le bicarbonate s'ils sont utilisés en combinaison,
- la méthode comprend en outre une étape préalable de prise d'empreinte d'une ou plusieurs dents et de réalisation du corps pour qu'il ait une forme complémentaire et des dimensions correspondant, à l'aspect lingual ou palatine, et/ou la surface occlusale, et/ou la surface vestibulaire de la ou des dents.

### Brève description des figures

La figure 1 est une représentation schématique d'une vue de dessus d'une première arcade dentaire sans dispositif selon l'invention.
La figure 2 est une représentation schématique d'une vue de dessus d'un premier mode de réalisation du dispositif selon l'invention.
La figure 3 est une représentation schématique d'une vue de dessus du dispositif de la figure 2, mis en place sur l'arcade dentaire représentée à la figure 1.
La figure 4 est une représentation schématique d'une vue de dessus d'une seconde arcade dentaire sans dispositif selon l'invention.
La figure 5 est une représentation schématique d'une vue de dessus d'un second mode de réalisation du dispositif selon l'invention.
La figure 6 est une représentation schématique d'une vue de dessus du dispositif de la figure 5, mis en place sur l'arcade dentaire représentée à la figure 4.
La figure 7 est une représentation schématique d'une vue en coupe de la figure 6.
La figure 8 est une représentation schématique d'une vue en coupe de la surface extérieure du corps ou de la surface externe du revêtement poreux avant la création des pores, après la création des pores et après une abrasion.

### Description détaillée de l'invention

Le dispositif dentaire 1 extra-coronaire selon l'invention, est un dispositif externe aux dents, qui se fixe sur la surface vestibulaire, la surface linguale ou palatine d'une ou plusieurs dents. Il ne s'agit pas d'une couronne ou d'un implant prothétique. Il peut s'agir d'une facette, d'une attelle ou d'un bridge, à savoir une attelle comprenant en outre une couronne prothétique 3, ou prothèse, destinée à remplacer une dent manquante.

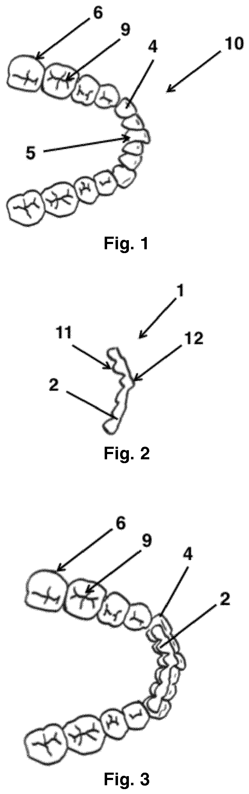

Le dispositif dentaire 1 extra-coronaire comprend un corps 2 extra-coronaire monobloc (figures 2, 5 et 7), qui n'est pas la couronne prothétique 3 dans le cas d'un bridge. Le corps 2 est sensiblement plein, c'est-à-dire non creux, sur au moins une partie de son épaisseur, fait, ou comprenant, un matériau comprenant, ou étant constitué, d'un polymère ou copolymère.

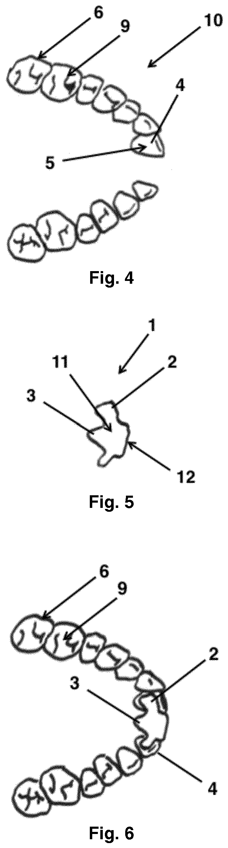

Dans le mode de réalisation dans lequel le dispositif dentaire est un bridge (figure 5), le corps 2 comprend, et associé à, une couronne prothétique 3, fait de tous matériaux ou mélanges de matériaux adéquats. De préférence, il est fait du même matériau que celui du dispositif dentaire 1.

Le corps 2 comprend, ou est fait, d'un polymère ou co-polymère, d'un polymère poly acrylique, poly méthacrylique ou poly(méthacrylate de méthyle) ou d'un ou plusieurs copolymère d'acide acrylique, d'acide méthacrylique et/ou de méthacrylate de méthyle. Il est obtenu en polymérisant une composition comprenant un ou plusieurs monomères polymérisables à base d'acrylate, étant de préférence choisis parmi l'acide acrylique, de l'acide méthacrylique, du méthacrylate de méthyle et leur mélange. De préférence, la composition du corps 2 comprend en outre un catalyseur de polymérisation, de préférence photo-activable, et/ou un accélérateur de polymérisation.

Le corps 2 polymérique, après polymérisation, ne comprend plus les monomères polymérisables ou de réactifs de départ, tous les composants ayant réagi ; néanmoins, comme dans toute réaction de polymérisation, il est possible que l'un ou l'autre des composants de la composition de polymérisation soit encore présent.

A titre d'exemple, le corps 2 est fait de poly-méthacrylate de méthyle, ou PMMA, et présente une dureté supérieure ou égale à 110 Vickers.

Le dispositif dentaire 1 comprend, à l'exception de la couronne prothétique 3 dans le cas d'un bridge, une porosité de sur une épaisseur comprise entre environ 0,1 et 0,8 mm, comprenant des pores dont la taille maximale, dans les trois dimensions, est comprise entre environ 10 *µ*m et 800 *µ*m, de préférence entre environ 10 *µ*m et 20 *µ*m, environ 10 *µ*m et 200 *µ*m, et environ 200 *µ*m à 800 *µ*m. Le dispositif dentaire 1 comprend des pores, qui sont ouverts vers l'extérieur pour ceux qui affleurent la surface exposée ou extérieure du dispositif dentaire 1, et fermés ou partiellement fermés pour ceux plus en profondeur. De préférence, les pores, ouverts ou non, sont interconnectés entre eux. De préférence, la porosité de surface, sur l'épaisseur en question, est comprise entre 20% et 60%.

Dans un premier mode de réalisation de l'invention, la porosité de surface est constitué par, et se situe sur, la surface externe du corps 2, dans l'épaisseur même du corps 2.

Dans un second mode de réalisation de l'invention, la porosité de surface est consistée par un revêtement poreux, recouvrant tout ou partie de la surface extérieure du corps 2, qui peut lui-même être poreux ou non poreux.

Qu'il s'agisse de la surface externe du corps 2 ou du revêtement poreux recouvrant la surface extérieure du corps 2, la porosité de surface est obtenue par la présence, dans la composition pour la réalisation du corps 2 et/ou celle pour la réalisation du revêtement poreux, d'une ou plusieurs charges porogènes, à savoir des charges aptes à créer des pores, de préférence par hydrolyse, et ce, de préférence, sans autres agents porogènes, ou moussant, autre que les charges minérales porogènes. Les charges porogènes ne réagissent pas toutes, par conséquent elles sont encore présentes, en tant que telles, dans le corps 2 dans son état final, sa surface externe, et/ou dans le revêtement poreux.

La composition pour la réalisation du corps 2 peut comprendre, outre les charges porogènes, d'autres charges par exemple des charges dites de renfort qui peuvent être minérales, permettant de modifier la dureté du corps 2 après polymérisation, et/ou un ou plusieurs pigments, qui peuvent également être des pigments minéraux.

Dans un autre mode de réalisation possible, la composition pour la réalisation du corps 2, et donc le corps 2, peut ne pas comprendre de charges porogènes, mais seulement des charges de renforts et/ou un ou plusieurs pigments minéraux.

Dans un autre mode de réalisation, la composition pour la réalisation du corps 2 ne comprend pas de charges porogènes, ni de charges de renfort, ni de pigments. Elle est exempte de toutes charges minérales, à l'exception d'éventuelles traces résiduelles pouvant être présent dans l'un ou l'autre des composants de la composition. Cette absence de charge présente l'avantage d'obtenir un matériau polymérisé plus facilement usinable, tout en état rigide et léger, avec une excellente tenue à la corrosion.

La composition pour la réalisation du corps 2 peut comprendre, outre un catalyseur de polymérisation, de préférence photo-activable, et/ou un accélérateur de polymérisation.

Le revêtement poreux selon l'invention comprend, ou est fait, d'un polymère ou copolymère, d'un polymère poly acrylique, poly méthacrylique ou poly(méthacrylate de méthyle) ou d'un ou plusieurs copolymère d'acide acrylique, d'acide méthacrylique et/ou de méthacrylate de méthyle. Il est obtenu à partir d'une composition comprenant un monomère polymérisable, ou un mélange de monomères polymérisables, et une ou plusieurs charges minérales porogènes, et ce, de préférence, sans autres agents porogènes, ou moussant, autre que les charges minérales porogènes.

De préférence, le ou les monomères polymérisables sont choisis parmi l'acide acrylique, de l'acide méthacrylique, du méthacrylate de méthyle et leur mélange.

De préférence, le ou les monomères polymérisables de la composition du revêtement sont identiques à ceux utilisés dans la composition du matériau du corps 2 qu'il recouvre.

De préférence, la composition à la base du revêtement poreux comprend en outre un catalyseur de polymérisation, de préférence photo-activable, et/ou un accélérateur de polymérisation.

Le revêtement poreux, après polymérisation, ne comprend plus les monomères polymérisables ou les réactifs de départ, tous les composants ayant réagi ; néanmoins, comme dans toute réaction de polymérisation, il est possible que l'un ou l'autre des composants de la composition de polymérisation soit encore présent.

La ou les charges minérales porogènes, de la composition de réalisation du corps 2 et/ou de la composition pour la réalisation du revêtement poreux, sont des charges permettant la formation de pores par un dégagement gazeux, de préférence générée par leur hydrolyse.

De préférence, la ou les charges minérales porogènes sont, ou comprennent, du carbonate et/ou du bicarbonate d'un sel d'un métal alcalin ou alcalinoterreux, de préférence d'un métal choisi parmi le sodium, le potassium ou le calcium.

Dans la composition pour la réalisation du corps 2, la ou les charges minérales porogènes représente(nt) 30 à 60 % en poids du poids total de la composition, de préférence entre 40 et 50 % en poids, de façon plus avantageusement environ 50 % en poids.

Dans la composition pour la réalisation du revêtement poreux, la ou les charges minérales porogènes représente(nt) 30 à 60 % en poids du poids total de la composition, de préférence entre 40 et 50 % en poids, de façon plus avantageusement environ 50 % en poids.

De préférence, la taille des charges minérales porogènes est comprise entre 10 et 100 *µ*m, de préférence entre 30 et 60 *µ*m.

La génération des pores peut se faire par l'action du ou des monomères à base d'acrylate, mais elle peut également se faire, ou être améliorée, par l'action d'un acide, par exemple de l'aide phosphorique, ou d'eau, présente dans la composition ou lors de la polymérisation, mais elle peut également se faire par une hydrolyse subséquente à la polymérisation de préférence réalisée à l'aide d'eau, de préférence une eau ayant une température supérieure à la température ambiante, de préférence comprise entre 50 et 100°C. Il peut également s'agir de vapeur d'eau.

L'emploi de charges minérales porogènes confère au dispositif dentaire 1 une porosité de surface particulière, des caractéristiques mécaniques et des propriétés de surface particulières, permettant une adhésion améliorée des ciments dentaires classiques au dispositif dentaire 1 et donc une fixation améliorée du dispositif dentaire 1 sur la ou les dents 4.

Dans un mode de réalisation particulier de l'invention, le dispositif dentaire 1 comprend une porosité de surface améliorée, par abrasion de la surface extérieure du corps 2 ou de la surface externe du revêtement poreux, de préférence à l'aide d'une solution à base d'érythriol, d'une composition à base d'Alumine (Al₂O₃), ou d'une composition comprenant des particules de carbonate et/ou bicarbonate d'un sel d'un métal alcalin ou alcalinoterreux, de préférence d'un métal choisi parmi le sodium, le potassium ou le calcium, le métal pouvant être identique ou différent entre le carbonate et le bicarbonate s'ils sont utilisés en combinaison. De préférence, il s'agit du même carbonate et/ou bicarbonate que celui présent dans la composition à la base du revêtement. La composition d'abrasion à base de carbonate et/ou bicarbonate peut comprendre en outre de l'oxyde d'alumine et de la silice, et comprend de l'eau et/ou de l'air pour permettre sa propulsion par des dispositifs classiques dans le domaine dentaire, comme par exemple un aéropolisseur.

Les charges minérales porogènes du corps 2 ou du revêtement poreux n'ayant pas réagies lors de la polymérisation, ou lors d'une hydrolyse subséquente, et ainsi exposées, régissent et permettent la création de nouveaux pores de surface et/ou l'agrandissement des pores existant, par un rinçage du revêtement polymérisé à l'aide d'eau, de préférence une eau ayant une température supérieure à la température ambiante, de préférence comprise entre 50 et 100°C. Il peut également s'agir de vapeur d'eau.

Cette porosité de surface améliorée présente l'avantage de conférer au dispositif dentaire 1 des propriétés de surface améliorée, permettant une meilleure adhésion du ciment dentaire au dispositif dentaire 1 et donc une meilleure fixation du dispositif dentaire 1 sur la ou les dents 4.

A titre d'exemples, une première série de dispositifs dentaires 1 selon l'invention (Da) a été réalisé en recouvrant un corps 2 en PMMA (GC Dental Products Corporation/ Kasugai, Aichi, Japon) à l'aide d'une composition de revêtement à base de méthacrylate de méthyle sur lesquels a été déposé un revêtement à base d'une composition de méthacrylate de méthyle en présence d'environ 50 % en poids de bicarbonate de soude (NaHCO₃) ayant une taille moyenne de particules d'environ 50 à 120 *µ*m, en tant que charges porogènes, mais sans autres charges minérales. Une seconde série de dispositifs dentaires 1 selon l'invention (Db) a été réalisé de la même manière mais avec une composition de revêtement comprenant environ 50 % en poids de carbonate de calcium (CaCO₃), ayant une taille moyenne de particules de 20 à 50 *µ*m, en tant que charges porogènes, et sans autres charges minérales. La taille des pores de surface présents sur la surface externe de leur corps 2 respectif a été mesurée, et comparée, à celle d'un dispositif dentaire témoin (D1 à D3) fait en PMMA, non recouvert d'un revêtement poreux, et dont la composition de départ ne comprend ni charges porogènes, ni autres charges minérales, pour différents traitements post-polymérisation. Les résultats sont donnés dans le tableau 1.

**Tableau 1 : Taille des pores**

| Echantillons | Traitements post polymérisation | | |
|---|---|---|---|
| | Aucun | Abrasion de la surface externe par des particules de NaHCO₃ | Abrasion de la surface externe par Al₂O₃ (particules de 50*µ*m) puis hydrolyse aqueuse |
| D1 | 0 | | |
| D2 | | 0 | |
| D3 | | | 0 à 80 *µ*m |
| Da1 | 10 à 20 *µ*m | | |
| Da2 | | 200 à 600 *µ*m | |
| Da3 | | | 200 à 800 *µ*m |
| Db1 | 10 *µ*m | | |
| Db2 | | 10 à 200 *µ*m | |
| Db3 | | | 10 à 200 *µ*m |

La mesure de la taille des pores, dans les trois dimensions, a été effectuée à l'aide d'un microscope Biolux Touch (Bresser GmbH, Rehe, Allemagne), avec un agrandissement de 350 fois, et un étalonnage au moyen d'une lame micrométrique (Bresser GmbH, Rehe, Allemagne) graduée de 0,1 à 0,01 mm. La taille des pores dans leur profondeur, a été mesurée de la même manière, mais couche par couche, en abrasant les échantillons, inclus dans un support époxy (Resin SRL, Arcola, Italie), à l'aide de disques abrasifs de granulométrie décroissante (Sof Lex ™ Pop on, 3M, Saint Paul, Minnesota USA).

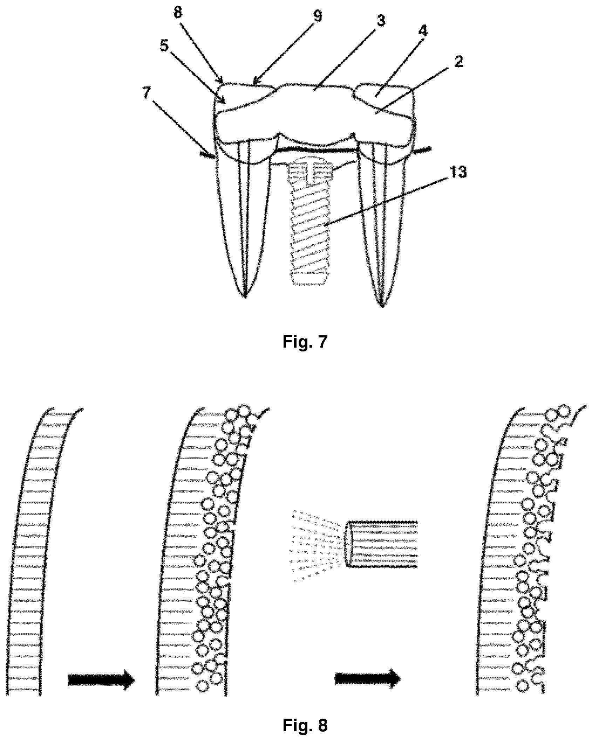

Il apparaît que l'abrasion, à l'aide de particules de bicarbonate de soude, des charges porogènes de la surface externe du revêtement recouvrant le corps 2 du dispositif dentaire 1, réalisée subséquemment à la polymérisation, augmente sensiblement la taille des pores (figure 8). Les charges porogènes, n'ayant pas réagi lors de la polymérisation, réagissent et permettent la génération de nouveaux pores ou leur agrandissement. Il en est de même pour une abrasion par des particules d'Alumine, une abrasion qui présente une porosité améliorée par rapport à une abrasion avec la composition comprenant des particules de bicarbonate de soude.

Des résultats comparables ont été obtenus pour des corps 2 réalisés à l'aide d'une composition de méthacrylate de méthyle et du bicarbonate de soude ou carbonate de calcium dans les mêmes proportions que celles pour le revêtement poreux.

De préférence, le corps 2 ou le revêtement poreux, est ou sont recouverte(s) d'un ciment d'adhésion, qui présente l'avantage d'une élimination aisée de tout excédent, sur le dispositif dentaire 1 et/ou sur les dents 4, à l'aide de moyens traditionnels, comme par exemple par l'emploi d'ultra-sons. De plus, un tel ciment présente l'avantage de permettre une bonne adhésion aux dents 4, sans pour autant que cette adhésion soit irréversible. Au contraire, par l'emploi de dispositifs classiques, comme un arrache couronne, il est possible de créer une fracture adhésive du ciment à l'interface avec le corps 2 du dispositif dentaire 1, notamment grâce à la flexion dudit corps 2.

De préférence, le ciment d'adhésion est un verre ionomère, par exemple celui connu sous la marque de fabrique Fuji 1, de GC Dental Products Corporation/ Kasugai, Aichi, Japon, ou un adhésif photo polymérisable, par exemple à base de méthacryloxy-déca-ethyl phosphate (MDP) comme par exemple le GC Ortho Connect Flow de GC Dental Products Corporation/ Kasugai, Aichi, Japon.

Les propriétés d'adhésion de différents dispositifs dentaires 1 selon l'invention, comprenant le revêtement poreux, et recouverts de l'adhésif photo polymérisable GC Ortho Connect Flow, ont été étudiées pour différentes charges porogènes, différentes proportions de charges porogènes et différents traitements post polymérisation. Ces propriétés d'adhésion sont évaluées en mesurant la force d'arrachement (mesurée en Newton) nécessaire pour séparer les dispositifs dentaires 1 d'un substrat de même nature et de même composition que le dispositif dentaire à tester correspondant (Tableaux 2 et 3).

Les revêtements poreux à base de méthacrylate de méthyle en présence de 50 % (poids/poids) de bicarbonate de soude (NaHCO₃) de la série Da du tableau 1 (Da1 à Da3) ont été comparés à des dispositifs dentaires dont la composition du revêtement poreux comprend du bicarbonate de calcium (NaHCO₃) ayant la même taille de particules, mais à différentes concentrations : 40 % en poids pour la série Da4 à Da6, 54% (poids/poids) pour la série Da7 et Da8, et 58% (poids/poids) pour la série Da9 et Da10. De même, les dispositifs dentaires 1 comprenant un revêtement poreux à base de méthacrylate de méthyle, en présence de 50 % en poids de carbonate de soude (CaCO₃) de la série Db du tableau 1 (Db1 à Db3) ont été comparés à des dispositifs dentaires dont la composition du revêtement poreux comprend du carbonate de soude (CaCO₃) ayant la même taille de particules, mais à différentes concentrations : 54 % en poids pour l'échantillon Db4 et 58 % en poids pour l'échantillon Db5.

**Tableau 2 : Mesure de force d'arrachement**

| Echantillons | Traitements post polymérisation | | |
|---|---|---|---|
| | Aucun | Abrasion de la surface externe avec des particules de NaHCO₃ | Abrasion de la surface externe avecAl₂O₃ (particules de 50*µ*m) puis hydrolyse aqueuse |
| D1 | 20 ± 3N | | |
| D2 | | 24 ± 6N | |
| D3 | | | 117± 18N |
| Da1 | 49 ± 20 N | | |
| Da2 | | 218 ± 31 N | |
| Da3 | | | 167 ± 20 N |
| Da4 | 46 ± 20 N | | |
| Da5 | | 175 ± 19 N | |
| Da6 | | | 149 ± 20 N |
| Da7 | 47 ± 16 N | | |
| Da8 | | 101 ± 11 N | |
| Da9 | 18 ± 7 N | | |
| Da10 | | 68 ± 16 N | |
| Db1 | 23 ± 8N | | |
| Db2 | | 97 ± 32 N | |
| Db3 | | | 111 ± 33 N |
| Db4 | 121 ± 33 N | | |
| Db5 | 54 ± 17 N | | |

Ensuite pour un même traitement post polymérisation (une abrasion par des particules de bicarbonate de soude), des dispositifs dentaires comprenant des revêtements poreux à base de différentes charges porogènes minérales, dans en même proportion (50 % en poids), ont été évalués et comparés au dispositif dentaire 1 Da2 dont la composition de revêtement comprend 50 % en poids de bicarbonate de soude (NaHCO₃), et à Db2 dont la composition de revêtement comprend 50 % en poids de carbonate de calcium (CaCO₃) (Tableau 3).

**Tableau 3 : Mesure de force d'arrachement**

| Echantillons | Nature des charges porogènes | Taille maximale des charges porogènes | Hydrolyse aqueuse |
|---|---|---|---|
| Da2 | NaHCO₃ | 90*µ*m | 218 ± 31 N |
| Db2 | CaCO₂ | 50*µ*m | 97 ± 32 N |
| Dc | NaCl | 90*µ*m | 223 ± 17 N |
| Dd | MgSO₄ | 90*µ*m | 191 ± 31 N |
| De | CaCl₂ | 100*µ*m | 235 ± 33 N |

Le NaHCO₃ est commercialisé sous la marque de fabrique connue sous Starwax de la société Brunel SAS (Wasquehal, France), le CaCO₃ est commercialisé sous la marque de fabrique connue Prophy pearls de la société Kaltenbach & Voigt GmbH (Biberach, Allemagne), le NaCl est du sel alimentaire commercialisé par la société La Baleine (Clichy, France), le MgSO₄ est commercialisé par la société Health Leads (Ceredigion, Grande Bretagne), et le CaCl₂ est commercialisé par la société Kalys Gastronomie (Bernin, France).

Il apparaît, de façon générale, que la présence de la porosité de surface améliore les propriétés d'adhésion des dispositifs dentaires 1 selon l'invention comparés avec les dispositifs dentaires témoins non poreux (D1 et D2). Ensuite, il apparaît que l'adhésion des dispositifs dentaires 1 selon l'invention est améliorée par un traitement post-polymérisation permettant la création de nouveaux pores de surface et/ou l'agrandissement des pores existant, qu'il s'agisse d'une abrasion par des particules de bicarbonate de soude ou d'une abrasion à l'aide de particules d'Alumine (Al₂O₃). La nature des charges minérales porogènes employées ne semble pas avoir d'influence drastique. Toutefois, les meilleurs résultats semblent être obtenus avec du chlorure de sodium (NaCl), du chlorure de calcium (CaCl₂) et du bicarbonate de soude (NaHCO₃). Même si le carbonate de calcium (CaCO₂) présente une bonne adhésion comparée aux témoins, il semble être moins la charge porogène testée la moins efficace, probablement du fait de la taille de ces particules (50 *µ*m), comparée à la taille moyenne (90 *µ*m) des autres charges porogènes. Enfin, la proportion de charges porogènes minérales dans le dispositif dentaire 1 selon l'invention s'avère influencer les propriétés d'adhésion des dispositifs dentaires 1, un optimum semblant se situer aux environs de 50 % en poids.

De préférence, une partie du corps 2, ou la ou les parties du corps 2 ne présentant pas de porosité de surface, éventuellement tout ou partie de la couronne prothétique 3 du dispositif dentaire 1 s'il s'agit d'un bridge, sont recouvertes d'un revêtement de protection non poreux, ayant toute composition et épaisseur adéquate. De préférence, le revêtement de protection est obtenu à partir d'une composition comprenant un monomère polymérisable, ou un mélange de monomères polymérisables, à base d'acrylate, de préférence choisis parmi l'acide acrylique, de l'acide méthacrylique, du méthacrylate de méthyle et leur mélange, sans charges minérales porogènes.

De préférence, le dispositif dentaire 1 est adapté à la dentition du patient, et apte à adhérer à au moins une face d'au moins une dent 4, de préférence de deux dents 4, avantageusement d'une pluralité de dents 4.

Le corps 2 du dispositif dentaire 1 peut recouvrir toute la ou les dents 4 sur laquelle ou lesquelles il se positionne. Toutefois, pour des raisons esthétiques il est préférable que le corps 2 ne recouvre qu'une partie de la dent 4 ou des dents 4, avantageusement une face 5, linguale ou palatine, de la ou des dents 4, ou vestibulaire 6, depuis la gencive 7 jusqu'au bord occlusal 8 ou éventuellement la surface occlusale 9.

De préférence, l'attelle ou le bridge sont positionnés légèrement sous le bord occlusal 8 pour permettre le respect de la phonation et optimiser le confort de mastication.

Le dispositif dentaire 1 peut se fixer sur la surface occlusale 9, donc la surface supérieure, de la dent 4 ou des dents 4. Toutefois, pour des raisons esthétiques, le dispositif dentaire 1 se fixe, de préférence, sur la face 5 linguale ou palatine, à savoir sur la surface arrière, de la dent 4 ou des dents 4.

De préférence, le dispositif dentaire 1 peut prendre une forme sensiblement longitudinale et horizontale, et adopter la forme de l'arcade dentaire 10 (figures 1 et 3 ou figures 4 et 6), ou de la portion de l'arcade dentaire 10, ou de la dent 4, sur laquelle il se fixe, ou il peut adopter une forme qui en est complémentaire.

De préférence, dans le mode de réalisation d'un bridge (figure 5), la couronne prothétique 3 reprend l'aspect et les dimensions de la dent 4 qu'il doit remplacer ou possède une forme et des dimensions compatibles.

De préférence, le dispositif dentaire 1 comprend au moins une première face 11 dite linguale ou palatine, destinée à faire face à la langue ou au palet du patient, et une seconde face 12, opposée à ladite surface linguale ou palatine. La première face 11 et/ou la seconde face 12 peut ou peuvent être en relief, à savoir comprendre un ou plusieurs motifs qui correspondent ou reprennent l'aspect lingual de la ou des dents 4 sur lesquelles la seconde face du dispositif dentaire 1 va venir en contact, ou être complémentaire, en forme et en dimensions, avec la forme de la face 5 linguale ou palatine de la ou des dents 4.

Le dispositif dentaire 1 est de préférence utilisé pour stabiliser une dent 4 ou groupe de dents 4 mobiles ou instables, pour prévenir, traiter ou stabiliser un déchaussement dentaire (figure 1), ou temporairement en prévision de la mise en place d'un implant 13 (figure 4).

La méthode de fabrication du dispositif dentaire 1 extra-coronaire selon l'invention comprend une étape de prendre, ou de réaliser, un corps 2 monobloc tel que décrit précédemment, comprenant ou fait d'un polymère ou copolymère, de préférence un polymère poly acrylique, poly méthacrylique ou poly(méthacrylate de méthyle) ou un ou plusieurs copolymère d'acide acrylique, d'acide méthacrylique et/ou de méthacrylate de méthyle, comprenant une porosité de surface sur une épaisseur comprise entre environ 0,1 et 0,8 mm, comprenant des pores dont la taille, dans les trois dimensions, est comprise entre environ 10 *µ*m et 800 *µ*m, de préférence entre environ 10 *µ*m et 20 *µ*m, environ 10 *µ*m et 200 *µ*m, et environ 200 *µ*m à 800 *µ*m. De préférence, la porosité de surface, sur l'épaisseur en question, est comprise entre 20% et 60%.

Dans un premier mode de réalisation la réalisation du corps 2 comprend une étape de prendre, ou réaliser, une composition pour la réalisation du corps 2 comprenant un ou plusieurs monomères polymérisables à base d'acrylate, étant de préférence choisis parmi l'acide acrylique, de l'acide méthacrylique, du méthacrylate de méthyle et leur mélange, et comprenant une ou plusieurs charges porogènes, générant des pores de préférence par hydrolyse, et éventuellement également des charges de renfort et/ou des pigments, éventuellement également un catalyseur de polymérisation, de préférence photo-activable, et/ou un accélérateur de polymérisation. La composition pour la réalisation du corps 2 est ensuite polymérisée, de préférence en présence d'un acide, par exemple de l'aide phosphorique, ou d'eau.

Dans un second mode de réalisation, la réalisation du corps 2 comprend une étape de prendre, ou réaliser, une composition pour la réalisation du corps 2 comprenant un ou plusieurs monomères polymérisables à base d'acrylate, étant de préférence choisis parmi l'acide acrylique, de l'acide méthacrylique, du méthacrylate de méthyle et leur mélange, mais sans charges porogènes, et une étape de polymérisation de ladite composition. Elle comprend en outre une étape de prendre, ou réaliser, une composition pour la réalisation d'un revêtement poreux comprenant un ou plusieurs monomères polymérisables à base d'acrylate, étant de préférence choisis parmi l'acide acrylique, de l'acide méthacrylique, du méthacrylate de méthyle et leur mélange, et comprenant une ou plusieurs charges porogènes, générant des pores de préférence par hydrolyse, et éventuellement également des charges de renfort et/ou des pigments, éventuellement également un catalyseur de polymérisation, de préférence photo-activable, et/ou un accélérateur de polymérisation, une étape de recouvrement de la tout ou partie de la surface extérieure du corps 2, de préférence sur tout ou partie de la surface du corps 2 destinée à venir en contact avec la ou les dents 4, à l'exception de la couronne prothétique 3 dans le cas d'un bridge, puis de polymérisation de ladite composition pour la réalisation du revêtement poreux sur ledit corps 2 de préférence en présence d'un acide, par exemple de l'aide phosphorique, ou d'eau.

Dans un troisième mode de réalisation, la réalisation du corps 2 comprend l'étape de prendre, ou réaliser, la composition pour la réalisation du corps 2 du premier mode de réalisation, l'étape de la polymériser afin d'obtenir le corps 2 ayant une surface extérieure poreuse, puis de prendre, ou réaliser, la composition pour la réalisation d'un revêtement poreux du second mode de réalisation, d'en revêtir tout ou partie de la surface externe du corps 2, de préférence sur tout ou partie de la surface du corps 2 destinée à venir en contact avec la ou les dents 4, à l'exception de la couronne prothétique 3 dans le cas d'un bridge, puis de la polymériser.

Quelque soit le mode de réalisation, les proportions de charges minérales porogènes représente(nt) en poids entre 30 et 60 % du poids total de la composition pour la réalisation du corps 2 et de la composition pour la réalisation du revêtement poreux, de préférence entre 40 et 50 % en poids, de façon plus avantageusement environ 50 % en poids.

De préférence, la réalisation du dispositif dentaire 1, et donc_du corps 2 monobloc comprend une étape préalable de prise d'empreinte de l'ensemble des dents 4 du patient, ou de la dent 4 ou des dents 4, avantageusement de l'arcade dentaire 10 ou portion d'arcade dentaire 10, sur laquelle ou lesquelles le dispositif dentaire 1 doit être appliqué. Cette étape peut être réalisée par l'emploi d'un produit classique de prise d'empreinte, de l'alginate par exemple, puis la réalisation d'un modèle en plâtre, avec éventuellement une modélisation informatique, en trois dimensions, du modèle en plâtre. Toutefois, de préférence, la prise d'empreinte se fait par la numérisation des dents 4 par une caméra optique intra-buccale et leur modélisation informatique.

Cette prise d'empreinte permet de déterminer la forme et les dimensions du dispositif dentaire 1 extra-coronaire, avant sa confection, une confection qui peut se faire soit par impression en trois dimensions ou par usinage d'un bloc d'un matériau comprenant, ou étant constitué, d'un polymère ou copolymère thermoplastique, tel que décrit précédemment.

Dans le mode de réalisation dans lequel le dispositif dentaire 1 extra-coronaire est un bridge, une couronne prothétique 3 est fournie en tant qu'élément distinct, puis fixé, de préférence de façon irréversible, au corps 2 monobloc, ou bien la couronne prothétique 3 fait partie intégrante du corps 2 monobloc et elle est réalisée de façon concomitante lors de la fabrication du corps 2 monobloc.

Le corps 2 monobloc du dispositif dentaire 1, avec ou sans couronne prothétique 3, peut être ensuite placé à son emplacement prévu dans la bouche, afin de s'assurer de la bonne réalisation de sa confection et de son bon positionnement futur. Si nécessaire, il peut être rebasé jusqu'à ce qu'il se positionne parfaitement.

Dans le mode de réalisation dans lequel la porosité de surface est obtenue à l'aide d'un revêtement poreux, la méthode selon l'invention peut comprendre une étape, de repositionnement, juste après l'application de la composition à la base du revêtement, du dispositif dentaire 1, à sa position prévue, dans la bouche du patient, la mise en contact avec la ou les dents 4, et le maintien dans cette position le temps que la composition à la base du revêtement polymérise et que tout ou partie des charges porogènes forme des pores dans l'épaisseur du revêtement, mais également à sa surface. Cela permet de réduire l'ensemble des erreurs dimensionnelles grâce à la déformation du revêtement qui est encore malléable.

Les excès de revêtement poreux sont, de préférence, éliminés.

De préférence, et afin d'accentuer les porosités en surface extérieure du corps 2 ou celle du revêtement poreux, de préférence celles devant entrer en contact avec la ou les dents 4 du patient, la méthode peut comprendre en outre une étape post-polymérisation d'hydrolyse de la surface externe du corps 2 ou de la surface externe du revêtement recouvrant le corps 2, mise en œuvre par l'action d'un acide, tel que par exemple de l'acide phosphorique, ou bien d'eau ayant une température supérieure à la température ambiante, de préférence comprise entre 50 et 100°C, ou de vapeur d'eau. L'étape post-polymérisation peut également consister en une abrasion réalisée, de préférence à l'aide d'une solution à base d'érythriol, d'une composition à base d'Alumine (Al₂O₃), ou d'une composition comprenant des particules de carbonate et/ou bicarbonate d'un sel d'un métal alcalin ou alcalinoterreux, de préférence d'un métal choisi parmi le sodium, le potassium ou le calcium, le métal pouvant être identique ou différent entre le carbonate et le bicarbonate s'ils sont utilisés en combinaison. De préférence, il s'agit du même carbonate et/ou bicarbonate que celui présent dans la composition à la base du revêtement, et pouvant comprendre en outre du phosphate de calcium et de la silice. Les charges ainsi exposées à la surface génèrent, sous l'action d'eau, de préférence chaude, des porosités de surface.

Dans un mode de réalisation particulier de l'invention, cette abrasion est réalisée à l'aide d'un aéropolisseur.

Un ciment, de préférence verre ionomère, ou un adhésif photo polymérisable, est appliqué sur le revêtement poreux recouvrant la ou les parties du corps 2 du dispositif dentaire 1 destinée à entrer en contact avec la ou les dents 4, de préférence, juste avant la mise en place du dispositif dentaire 1 dans la bouche.

De préférence, la méthode comprend en outre une étape de prendre ou préparer une composition de revêtement de protection, avantageusement comprenant un monomère polymérisable, ou un mélange de monomères polymérisables, à base d'acrylate, de préférence choisis parmi l'acide acrylique, de l'acide méthacrylique, du méthacrylate de méthyle et leur mélange, sans charges minérales porogènes, et une étape d'application de cette composition sur tout ou partie du corps 2, ou sur tout ou partie du corps 2 qui n'est pas recouvert du revêtement poreux selon l'invention et éventuellement également sur tout ou partie de la couronne prothétique 3.

Le dispositif dentaire 1 est positionné, et fixé, sur une ou plusieurs dents 4, de préférence sur leur surface occlusale 9 ou sur leur surface 6 linguale et/ou palatine, de la gencive 7 jusqu'au bord occlusal 8.

Le positionnent correct du dispositif dentaire 1 dans la bouche peut être éventuellement vérifié par la prise d'un image numérique ou optique intra-buccale.

S'agissant d'un dispositif dentaire 1 temporaire ou semi-permanent, le descellement du dispositif dentaire 1 se fait aisément en créant une fracture adhésive du ciment à l'interface avec le dispositif dentaire 1, par l'emploi par exemple d'un arrache couronne, et les excès de ciment sur la ou les dents 4 pouvant être éliminés aux ultra-sons ou à l'aide de fraises à finir, qui sont dédiées à cet effet.

## Revendications

1. Dispositif dentaire (1) extra-coronaire comprenant un corps (2) monobloc fait d'un polymère ou co-polymère, obtenu en polymérisant une composition comprenant un monomère polymérisable à base d'acrylate, ledit dispositif dentaire (1) comprenant une porosité de surface, sur une épaisseur comprise entre 0,1 et 0,8 mm, ladite porosité comprenant des pores, dont la taille est comprise entre 10 *µ*m et 800 *µ*m, sur ladite épaisseur.

2. Le dispositif dentaire (1) selon la revendication 1, dans lequel la porosité de surface comprend des pores dont la taille est comprise entre 10 *µ*m et 20 *µ*m, 10 *µ*m et 200 *µ*m ou 200 *µ*m et 800 *µ*m.

3. Le dispositif dentaire (1) selon la revendication 2, dans lequel la porosité de surface est comprise entre 20 à 60%.

4. Le dispositif dentaire (1) selon l'une quelconque des revendications précédentes, dans lequel la porosité de surface est constitué par la surface externe du corps (2) dudit dispositif dentaire (1), ou est constituée par un revêtement poreux recouvrant ladite surface externe dudit corps (2).

5. Le dispositif dentaire (1) selon l'une quelconque des revendications précédentes, dans lequel le corps (2) est fait d'un polymère ou co-polymère, obtenu en polymérisant une composition comprenant un monomère polymérisable choisis parmi l'acide acrylique, de l'acide méthacrylique, du méthacrylate de méthyle, et leur mélange.

6. Le dispositif dentaire (1) selon l'une quelconque des revendications 4 ou 5, dans lequel le revêtement poreux est fait d'un polymère ou co-polymère, obtenu en polymérisant une composition comprenant un monomère polymérisable choisis parmi l'acide acrylique, de l'acide méthacrylique, du méthacrylate de méthyle, et leur mélange.

7. Le dispositif dentaire (1) selon l'une quelconque des revendications précédentes, comprenant en outre un ciment d'adhésion recouvrant tout ou partie de la porosité de surface.

8. Le dispositif dentaire (1) selon l'une quelconque des revendications précédentes, ayant une forme complémentaire et des dimensions correspondant, à l'aspect lingual ou palatine, et/ou la surface occlusale (8), et/ou la surface vestibulaire (6) de la ou des dents (4) sur laquelle ou lesquelles ledit dispositif dentaire (1) va se fixer.

9. Le dispositif dentaire (1) selon l'une quelconque de revendications précédentes pour prévenir, traiter ou stabiliser un déchaussement dentaire, ou pour participer temporairement à la mise en place d'un implant (13).

10. Méthode de réalisation d'un dispositif dentaire (1) extra-coronaire comprenant un corps (2) extra-coronaire monobloc comprenant des porosités de surface, la méthode comprenant l'étape de :
- prendre ou réaliser, un corps (2) extra-coronaire monobloc à base d'un polymère ou copolymère thermoplastique, comprenant une porosité de surface sur une épaisseur comprise entre environ 0,1 et 0,8 mm, comprenant des pores, dont la taille est comprise entre 10 *µ*m et 800 *µ*m, sur ladite épaisseur.

11. La méthode selon la revendication 10 dans laquelle la réalisation du corps (2) extra-coronaire comprend les étapes de :
- prendre, ou réaliser, une composition pour la réalisation du corps (2) comprenant un ou plusieurs monomères polymérisables à base d'acrylate, et comprenant une ou plusieurs charges porogènes, ou ne comprenant pas de charges porogènes,
- prendre, ou réaliser, une composition pour la réalisation d'un revêtement poreux comprenant un ou plusieurs monomères polymérisables à base d'acrylate et comprenant une ou plusieurs charges porogènes,
- polymériser ladite composition pour la réalisation du corps (2) comprenant des charges porogènes, afin d'obtenir un corps (2) dont la surface extérieur est poreuse, ou polymériser ladite composition pour la réalisation du corps (2) ne comprenant pas de charges porogènes afin d'obtenir un corps (2) dont la surface extérieure n'est pas poreuse,
- recouvrir à l'aide de ladite composition pour la réalisation d'un revêtement poreux, le corps (2) dont la surface extérieure est poreuse, ou le corps (2) dont la surface extérieure n'est pas poreuse, puis polymériser ladite composition pour la réalisation d'un revêtement poreux.

12. La méthode selon l'une quelconque des revendications 10 ou 11, dans laquelle la méthode comprend en outre une étape d'accentuer la porosité de la surface extérieure du corps (2), ou celle du revêtement poreux, par une hydrolyse de ladite surface extérieure, ou par une étape d'abrasion de ladite surface extérieure à l'aide d'une solution à base d'érythriol, d'une composition à base d'Alumine (Al₂O₃), ou à base de carbonate et/ou bicarbonate d'un sel d'un métal alcalin ou alcalinoterreux, de préférence d'un métal choisi parmi le sodium, le potassium ou le calcium, le métal pouvant être identique ou différent entre le carbonate et le bicarbonate s'ils sont utilisés en combinaison.

13. La méthode selon l'une quelconque des revendications 10 à 12, comprenant en outre une étape préalable de prise d'empreinte d'une ou plusieurs dents (4) et de réalisation du corps (2) pour qu'il ait une forme complémentaire et des dimensions correspondant, à l'aspect lingual ou palatine, et/ou la surface occlusale (8), et/ou la surface vestibulaire (6) de la ou des dents (4).

## Patentansprüche

1. Extrakoronare Dentalvorrichtung (1), umfassend einen Einblock-Körper (2) aus einem Polymer oder Copolymer, erhalten durch Polymerisieren einer Zusammensetzung, die ein polymerisierbares Monomer auf der Basis von Acrylat umfasst, wobei die Dentalvorrichtung (1) eine Oberflächenporosität über eine Dicke zwischen 0,1 und 0,8 mm umfasst, wobei die Porosität Poren umfasst, deren Größe über die Dicke zwischen 10 *µ*m und 800 *µ*m beträgt.

2. Dentalvorrichtung (1) nach Anspruch 1, wobei die Oberflächenporosität Poren umfasst, deren Größe zwischen 10 *µ*m und 20 *µ*m, 10 *µ*m und 200 *µ*m oder 200 *µ*m und 800 *µ*m beträgt.

3. Dentalvorrichtung (1) nach Anspruch 2, wobei die Oberflächenporosität zwischen 20 bis 60 % beträgt.

4. Dentalvorrichtung (1) nach einem der vorangehenden Ansprüche, wobei die Oberflächenporosität von der äußeren Oberfläche des Körpers (2) der Dentalvorrichtung (1) gebildet ist oder von einer porösen Beschichtung gebildet ist, welche die äußere Oberfläche des Körpers (2) bedeckt.

5. Dentalvorrichtung (1) nach einem der vorangehenden Ansprüche, wobei der Körper (2) aus einem Polymer oder Copolymer ist, erhalten durch Polymerisieren einer Zusammensetzung, die ein polymerisierbares Monomer umfasst, ausgewählt aus der Acrylsäure, der Methacrylsäure, dem Methylmethacrylat und ihrem Gemisch.

6. Dentalvorrichtung (1) nach einem der Ansprüche 4 oder 5, wobei die poröse Beschichtung aus einem Polymer oder Copolymer ist, erhalten durch Polymerisieren einer Zusammensetzung, die ein polymerisierbares Monomer umfasst, ausgewählt aus der Acrylsäure, der Methacrylsäure, dem Methylmethacrylat und ihrem Gemisch.

7. Dentalvorrichtung (1) nach einem der vorangehenden Ansprüche, umfassend ferner einen Haftzement, der die gesamte oder einen Teil der Oberflächenporosität bedeckt.

8. Dentalvorrichtung (1) nach einem der vorangehenden Ansprüche, die eine komplementäre Form und Abmessungen hat, die dem lingualen oder palatinalen Aussehen und/oder der okklusalen Oberfläche (8) und/oder der vestibulären Oberfläche (6) des oder der Zähne (4) entsprechen, auf dem oder denen die Dentalvorrichtung (1) befestigt wird.

9. Dentalvorrichtung (1) nach einem der vorangehenden Ansprüche zur Vorbeugung, Behandlung oder Stabilisierung eines Zahnfleischschwunds oder zur temporären Beteiligung an der Platzierung eines Implantats (13).

10. Verfahren zur Herstellung einer extrakoronaren Dentalvorrichtung (1), umfassend einen extrakoronaren Einblock-Körper (2), umfassend Oberflächenporositäten, wobei das Verfahren den Schritt umfasst:
- Bereitstellen oder Herstellen eines extrakoronaren Einblock-Körpers (2) auf der Basis eines thermoplastischen Polymers oder Copolymers, umfassend eine Oberflächenporosität über eine Dicke zwischen zirka 0,1 und 0,8 mm, umfassend Poren, deren Größe über die Dicke zwischen 10 *µ*m und 800 *µ*m beträgt.

11. Verfahren nach Anspruch 10, wobei die Herstellung des extrakoronaren Körpers (2) die Schritte umfasst:
- Bereitstellen oder Herstellen einer Zusammensetzung für die Herstellung des Körpers (2), umfassend ein oder mehrere polymerisierbare Monomere auf der Basis von Acrylat und umfassend einen oder mehrere porenbildende Füllstoffe oder umfassend keine porenbildenden Füllstoffe,
- Bereitstellen oder Herstellen einer Zusammensetzung für die Herstellung einer porösen Beschichtung, umfassend ein oder mehrere polymerisierbare Monomere auf der Basis von Acrylat und umfassend einen oder mehrere porenbildende Füllstoffe,
- Polymerisieren der Zusammensetzung, um den Körper (2) herzustellen, der porenbildende Füllstoffe umfasst, um einen Körper (2) zu erhalten, dessen äußere Oberfläche porös ist, oder Polymerisieren der Zusammensetzung, um den Körper (2) herzustellen, der keine porenbildenden Füllstoffe umfasst, um einen Körper (2) zu erhalten, dessen äußere Oberfläche nicht porös ist,
- Bedecken, mit Hilfe der Zusammensetzung für die Herstellung einer porösen Beschichtung, des Körpers (2), dessen äußere Oberfläche porös ist, oder des Körpers (2), dessen äußere Oberfläche nicht porös ist, dann Polymerisieren der Zusammensetzung für die Herstellung einer porösen Beschichtung.

12. Verfahren nach einem der Ansprüche 10 oder 11, wobei das Verfahren ferner einen Schritt der Akzentuierung der Porosität der äußeren Oberfläche des Körpers (2) oder der der porösen Beschichtung durch eine Hydrolyse der äußeren Oberfläche oder durch einen Schritt des Abriebs der äußeren Oberfläche mit Hilfe einer Lösung auf der Basis von Erythriol, einer Zusammensetzung auf der Basis von Aluminiumoxid (Al₂O₃) oder auf der Basis von Carbonat und/oder Bicarbonat eines Salzes eines alkalischen oder erdalkalischen Metalls, vorzugsweise eines Metalls, ausgewählt aus Natrium, Kalium oder Calcium umfasst, wobei das Metall zwischen dem Carbonat und dem Bicarbonat identisch oder unterschiedlich sein kann, wenn sie kombiniert verwendet werden.

13. Verfahren nach einem der Ansprüche 10 bis 12, umfassend ferner einen vorherigen Schritt des Abdrucknehmens von einem oder mehreren Zähnen (4) und der Herstellung des Körpers (2), damit er eine komplementäre Form und entsprechende Abmessungen hinsichtlich des lingualen oder palatinalen Aussehens und/oder der okklusalen Oberfläche (8) und/oder der vestibulären Oberfläche (6) des oder der Zähne (4) hat.

## Claims

1. Extracoronal dental device (1) comprising a one-piece body (2) made from a polymer or copolymer, obtained by polymerizing a composition comprising a polymerizable monomer based on acrylate, said dental device (1) comprising a surface porosity, over a thickness between 0.1 and 0.8 mm, said porosity comprising pores, the size of which is between 10 µm and 800 µm, over said thickness.

2. The dental device (1) according to claim 1, wherein the surface porosity comprises pores having a size between 10 µm and 20 µm, 10 µm and 200 µm or 200 µm and 800 µm.

3. The dental device (1) according to claim 2, wherein the surface porosity is between 20 to 60%.

4. The dental device (1) according to any one of the preceding claims, wherein the surface porosity is made up of the outer surface of the body (2) of said dental device (1), or is made up of a porous coating covering said outer surface of said body (2).

5. The dental device (1) according to any one of the preceding claims, wherein the body (2) is made up of a polymer or copolymer, obtained by polymerizing a composition comprising a polymerizable monomer selected from acrylic acid, methacrylic acid, methyl methacrylate, and mixtures thereof.

6. The dental device (1) according to any one of claims 4 or 5, wherein the porous coating is made from a polymer or copolymer, obtained by polymerizing a composition comprising a polymerizable monomer selected from acrylic acid, methacrylic acid, methyl methacrylate, and mixtures thereof.

7. The dental device (1) according to any one of the preceding claims, further comprising an adhesion cement covering all or part of the surface porosity.

8. The dental device (1) according to any one of the preceding claims, having a shape that is complementary and dimensions corresponding to the lingual or palatal aspect, and/or to the occlusal surface (8), and/or the vestibular surface (6) of the tooth or teeth (4) on which said dental device (1) will be attached.

9. The dental device (1) according to any one of the preceding claims for preventing, treating or stabilizing a dental loosening, or to participate temporarily in the placement of an implant (13).

10. Method for producing an extracoronal dental device (1) comprising a one-piece extracoronal body (2) comprising surface porosities, the method comprising the step of:
- taking or producing a one-piece extracoronal body (2) based on a thermoplastic polymer or copolymer, comprising a surface porosity over a thickness of between about 0.1 and 0.8 mm, comprising pores, the size of which is between 10 µm and 800 µm, over said thickness.

11. The method according to claim 10, wherein the production of the extracoronal body (2) comprises the following steps:
- taking or producing a composition for producing the body (2) comprising one or several polymerizable monomers based on acrylate, and comprising one or several pore-forming fillers, or not comprising pore-forming fillers,
- taking or producing a composition for making a porous coating comprising one or several polymerizable monomers based on acrylate and comprising one or several pore-forming fillers,
- polymerizing said composition to produce the body (2) comprising pore-forming fillers, in order to obtain a body (2) whose outer surface is porous, or polymerizing said composition in order to produce the body (2) not comprising pore-forming fillers in order to obtain a body (2) whose outer surface is not porous,
- covering, using said composition to produce a porous coating, the body (2) whereof the outer surface is porous, or the body (2) whereof the outer surface is not porous, then polymerizing said composition to produce a porous coating.

12. The method according to any one of claims 10 or 11, wherein the method further comprises a step for accentuating the porosity of the outer surface of the body (2), or that of the porous coating, by hydrolysis of said outer surface, or by a step for abrasion of said outer surface using a solution based on erythriol, a composition based on Alumina (Al₂O₃), or based on carbonate and/or bicarbonate of a salt of an alkali or alkaline earth metal, preferably a metal selected from sodium, potassium or calcium, the metal being able to be identical or different between carbonate and bicarbonate if they are used in combination.

13. The method according to any one of claims 10 to 12, further comprising a prior step for taking an imprint of one or several teeth (4) and producing the body (2) so that it has a shape that is complementary and dimensions corresponding to the lingual or palatal aspect, and/or the occlusal surface (8), and/or the vestibular surface (6) of the tooth or teeth (4).
